Europäisches Patentamt

European Patent Office

Office européen des brevets

(19)

(11) Veröffentlichungsnummer : **0 170 821**
**B1**

(12) **EUROPÄISCHE PATENTSCHRIFT**

(45) Veröffentlichungstag der Patentschrift :
15.03.89

(21) Anmeldenummer : 85107087.0

(22) Anmeldetag : 08.06.85

(51) Int. Cl.⁴ : **A 61 M 37/00**, A 61 F 13/02

(54) **Selbstklebendes Pflaster.**

(30) Priorität : 23.06.84 DE 3423293

(43) Veröffentlichungstag der Anmeldung :
**12.02.86 Patentblatt 86/07**

(45) Bekanntmachung des Hinweises auf die Patenterteilung : 15.03.89 Patentblatt 89/11

(84) Benannte Vertragsstaaten :
**AT BE CH DE FR GB IT LI NL SE**

(56) Entgegenhaltungen :
CH–A– 341 948
DE–A– 3 202 775

(73) Patentinhaber : **Beiersdorf Aktiengesellschaft
Unnastrasse 48
D-2000 Hamburg 20 (DE)**

(72) Erfinder : **Nick, Erich, Dr.
Eichhörnchenweg 2
D-2080 Pinneberg (DE)**
Erfinder : **Guse, Günter, Dr. Dipl.-Chem.
Liliencronstrasse 30
D-2000 Hamburg 73 (DE)**
Erfinder : **Asmussen, Bodo, Dr.
Dorotheenweg 1a
D-2071 Ammerbek (DE)**

EP 0 170 821 B1

**Beschreibung**

Die Erfindung betrifft ein Verfahren zur Herstellung eines selbstklebenden Pflasters mit separaten Wirkstoffsegmenten auf einem klebstoffbeschichteten Träger zur transdermalen Applikation.

Derartige Pflaster sind bekannt. So beschreibt die DE-OS 3 302 775 ein Adhäsions-Pflaster, bei dem auf einer Kleberschicht Wirkstoff-Segmente aufgetragen sind. Dabei befindet sich der Kleber auf einem Träger und das Ganze wird mit einer Abdeckung geschützt, die vor Gebrauch abgezogen wird.

Obgleich ein solches Pflaster wirtschaftlich herstellbar ist, weist es gravierende Nachteile auf, die es für die Praxis unzulänglich machen. Durch die dem System immanente Wechselwirkung zwisschen Kleber und Wirkstoff ist es einerseits erforderlich, sowohl den Kleber an die Galenik des Wirkstoffes anzupassen, als auch andererseits die Galenik des Wirkstoffs an den Kleber anzupassen. Zudem ist durch die Interaktion zwischen Wirkstoff und Kleber die erforderliche Lagerstabilität eines solchen Pflasters nicht zur erfüllen, so daß es den strengen behördlichen Anforderungen an die Registrierung als Arzneimittel nicht genügen kann.

Insbesondere bei längerer Lagerung unter erschwerten Bedingungen migriert Wirkstoff in den Kleber und umgekehrt migrieren Bestandteile des Klebers in die Wirkstoff-Formulierung, zumal es sich bei einem Kleber nicht um eine chemisch einheitliche Substanz sondern um ein Stoffgemisch handelt.

Aufgabe der Erfindung war es, hier Abhilfe zu schaffen, insbesondere ein Pflaster und ein Verfahren zu dessen Herstellung ohne die Nachteile des Standes der Technik zu entwickeln.

Aus der Literatur sind Verfahren zum Bedrucken von flächigen Trägern bekannt. So können im Siebdruck sowie im Tiefdruck präzise umrissene Flächen sicher bedruckt werden, sofern das zu bedruckende Material keine Unebenheiten aufweist oder sich während des Druckvorgangs verzieht. Es mußte aber angenommen werden, daß es unmöglich ist, beim Bedrucken einer unebenen Oberfläche eine hinreichende Druckpräzision zu erreichen (H. Klein. Adhäsion 1976, Heft 7, S. 185 ff, EP-OS 0 099 587). Eine mit einer Vielzahl von Trennfilmsegmeten bedruckte Pflasterbahn ist aber notgedrungen uneben und sollte infolgedessen in einem weiteren Druckdurchgang nicht mehr paßgenau konfektionierbar sein.

So war es überraschend und nicht vorherzusehen, daß es entgegen dem Vorurteil der einschlägigen Literatur möglich ist, in einem ersten Druckvorgang auf einen klebstoffbeschichteten Träger eine Vielzahl von Trennfilmsegmenten zu drucken und in einem zweiten Druckschritt auf die Trennfilmsegmente Wirkstoffsegmente aufzutragen.

Dieser Trennfilm besteht vorzugsweise aus einer Membran, die störende Wechselwirkungen zwischen Wirkstoffzubereitung und Kleberschicht ausschließt, also im Sinne der Funktion des erfindungsgemäßen Gegenstandes nicht-permeabel ist. In besonders geeigneter Weise ist dieser Trennfilm z. B. aus Polyvinylidenchlorid oder Copolymeren davon und auch das auf ihm angeordnete Wirkstoff-Segment aus jeweils einer Dispersion mit hohem Feststoffgehalt im Druckverfahren vom Typ Tiefdruck oder insbesondere Siebdruck aufgetragen.

Damit wird erfindungsgemäß erreicht, daß es zu einer Interaktion zwischen Wirkstoff und Kleber nicht kommen kann.

Die Zusammensetzung des Klebers braucht erfindungsgemäß keine Rücksicht mehr auf den Wirkstoff zu nehmen. Es können übliche Selbstklebemassen verwendet werden, insbesondere hautfreundliche derartige Massen, wie solche auf Acrylat-Basis. Zur Verankerung dieser Kleber auf dem Träger können gegebenenfalls Haftvermittler verwendet werden, die als Vorstrich aufgetragen werden.

Sollen als Klebemassen etwa elektronenstrahlvernetzte Massen verwendet werden, so wird die Vernetzung vorzugsweise durchgeführt, bevor die Wirkstoffzubereitung auf die Segmente des Trennfilms aufgebracht wird. Dies kann vorteilhaft in Linie erfolgen, wobei zunächst in an sich bekannter Weise der Kelbstoff auf den Träger beschichtet wird, dann im Siebdruckverfahren bzw. Tiefdruckverfahren die Segmente des Trennfilms aufgebracht werden, worauf vernetzt wird, um schließlich auf die Segmente des Trennfilms im Siebdruck die Wirkstoffzubereitung aufzutragen. Vorteilhaft ist es, wenn die Trennfilm-Segmente einen etwas größeren Durchmesser als die Wirkstoff-Punkte haben.

Die Wirkstoffzubereitung enthält filmbildende und andere geeignete pharmazeutische Hilfsstoffe, die dem Fixieren auf dem Trennfilm und der optimalen Wirkstoffdiffusion in die Haut dienlich sind.

Vorteilhaft sind die Wirkstoff-Segmente in regelmäßiger Folge angeordnet und haben einen Durchmesser bis zu etwa 5 000 µm, insbesondere 200-1 500 µm.

| | |
|---|---|
| Antihypertonika | Psychopharmaka |
| Antihypotonika | Antiasthmatika |
| Vasodilatantien | Antirheumatika |
| β-Blocker | Antiarythmika |
| Calciumantagonisten | Antihystaminika |
| Antiemetika | Hormone |
| Antitussiva | Antibiotika |
| Sedativa | Cytostatika |
| Analgetika | |

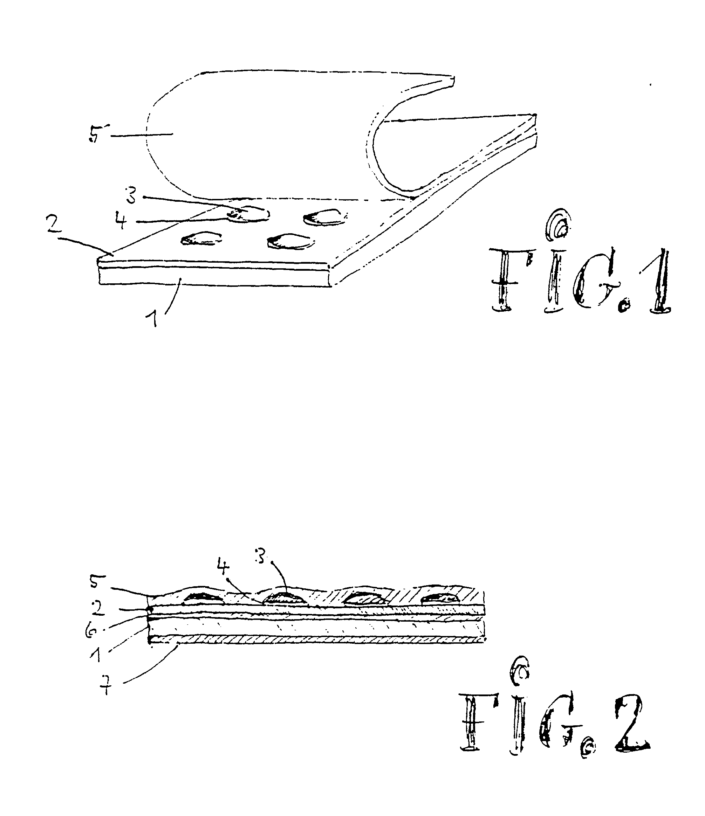

In den Fig. 1 und 2 wird die Erfindung anhand von Beispielen erläutert, ohne daß damit eine Einschränkung ausgesprochen werden soll. Vielmehr kann der Fachmann anhand seines Fachwissens Änderungen vornehmen, ohne den Rahmen der Erfindung zu verlassen.

Im einzelnen zeigt Fig. 1 einen Träger 1, auf dem sich eine Klebstoffschicht 2 befindet. Auf dieser wiederum befinden sich Segmente von Trennfilmen 4, auf denen Wirkstoff-Segmente 3 angeordnet sind. Diese wiederum sind mit einer Abdeckung 5 geschützt, wobei die Abdeckung 5 vor einer Verwendung abgezogen wird. In Fig. 2 befindet sich zwischen dem Träger 1 und der Klebstoffschicht 2 eine Schicht eines Haftvermittlers 6, um den Kleber auf dem Träger 1 besser zu verankern. Auf der anderen Seite des Trägers 1 befindet sich ein Decklack 7 und/oder eine aufgedampfte Metallschicht. Wirkstoff-Segmente 3, Trennfilm 4 und Abdeckung 5 sind ebenfalls dargestellt.

Beispiel 1

A. Herstellung einer Wirkstoffzubereitung

27,35 GT Hydroxypropylmethylcellulose (Pharmacoat 603) werden in ein Gemisch von 30 GT Isopropanol, 30 GT Wasser und 8,20 GT 1,2-Propylenglycol kalt eingerührt. Das Gemisch wird unter ständigem Rühren auf 50 °C erwärmt, bis eine klare Lösung entstanden ist. In diese werden 4,45 GT micronisiertes Moxonidin sorgfältig eingemischt.

B. Herstellung einer Trennfilmzubereitung

100 GT einer handelsüblichen wässrigen Polyvinylidenchlorid-Dispersion (Diofan 190 D) werden durch Zusatz von 2 GT einer 10 %igen wässrigen Lösung des Ammoniumsalzes einer Polyacrylsäure (Latekoll AS) auf eine Viscosität von etwa 100 Pas verdickt.

C. Vorbereitung der Trägerbahn

Zunächst wird eine Trägerbahn aus einseitig aluminisierter und hautfarben lackierter Polyethylenterephthalat-Folie von 15 μm Stärke (Hostaphan RN 15) mit einer Selbstklebemasse vollflächig beschichtet, so daß sich ein Trocken-Auftragsgewicht von etwa 35 g/m² ergibt. Gut geeignet ist beispielsweise eine Selbstklebemasse auf Acrylsäureester-Basis gemäß DE-PS 2 743 979.

D. Druckvorgang

Auf die getrocknete und vernetzte Selbstklebeschicht wird mit einem ersten rotierenden Siebdruckwerk bei einer Bahngeschwindigkeit von 20 m/Min. die Trennfilmzubereitung aufgetrager. Dabei wird ein Siebzylinder verwendet, der je Quadratzentimeter 200 Punkte von je 500 μm Basisdurchmesser in regelmäßiger Anordnung erzeugt. Die Auftragsmenge wird über die Rakeleinstellung so geregelt, daß flache, fest auf der Polyacrylatschicht haftende PVDC-Filmsegmente entstehen, die im getrockneten Zustand eine Dicke von etwa 20 μm besitzen. Die Trocknung erfolgt in einem Warmluft-Trockenkanal von 6 m Länge bei etwa 70 °C.

Ein zweites rotierendes Siebdruckwerk gleichen Typs druckt anschließend auf die PVDC-Filmsegmente passergenaub die Wirkstoffpunkte von je 400 μm Basisdurchmesser. Die Auftragsmenge wird über die Rakeleinstellung so geregelt, daß die kalottenförmigen Punkte im getrockneten Zustand eine Höhe von etwa 160 μm besitzen. Das entspricht einer Auftragsmenge von etwa 25 g Wirkstoffzubereitung je m² Träger. Hinter diesem zweiten Druckwerk folgt erneut eine Trockenstrecke des beschriebenen Typs und anschließend ein Kaschierwerk.

E. Konfektionierung

Die zweifach bedruckte, getrocknete und abgedeckte Materialbahn kann nun im Einzelpflaster beliebiger Größe aufgeteilt werden, je nach der erwünschten Wirkstoffdosierung. Wählt man beispielsweise eine Größe von 25 cm², so hat man auf dieser Fläche etwa 5 000 Wirkstoffpunkte entsprechend einem Gehalt von 7 mg Moxonidin [2-Methyl-4-chlor-6-methoxy-5-(2-imidazolin-2-yl)-amino-pyrimidin]. Diese Pflaster werden einzeln in ein diffusionsdichtes Primärpackmittel eingeschweißt, beispielsweise einen Flachbeutel aus PE/Alu/Papier-Verbundmaterial.

**Patentansprüche**

1. Verfahren zur Herstellung eines selbstklebenden Pflasters zur transdermalen Applikation im Siebdruck oder Tiefdruck, wobei man auf einem mit Klebstoff (2) beschichteten Träger (1) Trennfilm-Segmente (4) druckt, und dann auf jedes dieser Trennfilm-Segmente (4) jeweils ein Wirkstoff-Segment (3) druckt.

2. Verfahren nach Anspruch 1, dadurch gekennzeichnet, daß der Trennfilm (4) eine nicht-permeable Membran aus z. B. Polyvinylidenchlorid oder Copolymeren davon oder Polyvinylacetat ist.

3. Verfahren nach Anspruch 1 oder 2, dadurch gekennzeichnet, daß der Trennfilm (4) und die Wirkstoff-Segmente (3) aus Dispersionen mit hohem Feststoffehalt im Druckverfahren vom Typ Tiefdruck oder insbesondere Siebdruck aufgetragen sind.

4. Verfahren nach einem der Ansprüche 1-3, dadurch gekennzeichnet, daß sich auf den Wirkstoff-Segmenten (3) eine Abdeckung (5) befindet, ggf. auf der gegenüberliegenden Seite des Trägers (1) ein Decklack (7) befindet, und ggf. zwischen Träger (1) und Klebstoffschicht (2) ein Haftvermittler (6) befindet.

5. Verfahren nach einem der Ansprüche 1-4, dadurch gekennzeichnet, daß ein Teil der Wirkstoff-Segmente (3) einen Wirkstoff, der Rest einen anderen Wirkstoff enthält.

6. Verfahren nach einem der Ansprüche 1-5, dadurch gekennzeichnet, daß die Wirkstoff-Segmente (3) in regelmäßiger Folge angeordnet sind und einem Durchmesser von bis zu etwa 600 My haben, insbesondere 100-500 My.

7. Verfahren nach einem der Ansprüche 1-7, dadurch gekennzeichnet daß der Wirkstoff Moxonidin ist.

## Claims

1. Process for the production of a self-adhesive plaster for transdermal administration by screen printing or roller printing, entailing partition film segments (4) being printed on a support (1) coated with adhesive (2), and then in each case an active substance segment (3) being printed on each of theses partition film segments (4).

2. Process according to Claim 1, characterized in that the partition film (4) is a non-permeable membrane composed of, for example, polyvinylidene chloride or copolymers thereof or polyvinyl acetate.

3. Process according to Claim 1 or 2, characterized in that the partition film (4) and the active substance segments (3) are applied from dispersions having a high solids content in a printing process of the roller printing or, in particular, screen printing type.

4. Process according to one of Claims 1-3, characterized in that a covering (5) is located on the active substance segments (3), where appropriate a cover coating (7) is located on the opposite side of the support (1), and, where appropriate, an adhesion promoter (6) is located between the support (1) and adhesive layer (2).

5. Process according to one of Claims 1-4, characterized in that one part of the active substance segments (3) contains one active substance, and the remainder contains another active substance.

6. Process according to one of Claims 1-5, characterized in that the active substance segments (3) are arranged in regular sequence and have a diameter of up to about 600 mu, in particular 100-500 mu.

7. Process according to one of Claims 1-6, characterized in that the active substance is moxonidine.

## Revendications

1. Procédé de fabrication d'un pansement autoadhésif pour l'application transdermique par sérigraphie ou impression en creux, suivant lequel on imprime des segments de film de séparation (4) sur un support (1) revêtu d'un adhésif (2) et on imprime ensuite, sur chacun des segments de film de séparation (4), un segment de principe actif (3).

2. Procédé suivant la revendication 1, caractérisé en ce que le film de séparation (4) est une membrane non perméable, par exemple en poly(chlorure de vinylidène) ou en copolymères de celui-ci ou bien en poly(acétate de vinyle).

3. Procédé suivant la revendication 1 ou 2, caractérisé en ce que le film de séparation (4) et les segments de principe actif (3) sont appliqués au moyen de dispersions à haute teneur en solides par un procédé d'impression du type impression en creux ou en particulier impression sérigraphique.

4. Procédé suivant l'une quelconque des revendications 1 à 3, caractérisé en ce qu'une couverture (5) se trouve sur les segments de principe actif (3), un vernis de couverture (7) se trouve éventuellement sur la face opposée du support (1) et un intermédiaire d'adhérence (6) se trouve éventuellement entre le support (1) et la couche d'adhésif (2).

5. Procédé suivant l'une quelconque des revendications 1 à 4, caractérisé en ce que certains des segments de principe actif (3) contiennent un principe actif et les autres contiennent un autre principe actif.

6. Procédé suivant l'une quelconque des revendications 1 à 5, caractérisé en ce que les segments de principe actif (3) sont agencés en succession régulière et ont un diamètre s'élevant jusqu'à environ 600 μm et en particulier de 100 à 500 μm.

7. Procédé suivant l'une quelconque des revendications 1 à 6, caractérisé en ce que le principe actif est la monoxidine.

FIG.1

FIG.2